Europäisches Patentamt

**European Patent Office**

Office européen des brevets

(11) Veröffentlichungsnummer: **0 362 731**
**A2**

(12) **EUROPÄISCHE PATENTANMELDUNG**

(21) Anmeldenummer: 89118144.8

(51) Int. Cl.5: **A61K 9/16**

(22) Anmeldetag: 30.09.89

(30) Priorität: 01.10.88 DE 3833446

(43) Veröffentlichungstag der Anmeldung:
**11.04.90 Patentblatt 90/15**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

Anmelder: **HOECHST CELANESE**
**CORPORATION**
**Route 202-206 North**
**Somerville, N.J. 08876(US)**

(72) Erfinder: **Rittner, Siegbert, Dr.**
**Kornblumenweg 5**
**D-6082 Mörfelden-Walldorf(DE)**
Erfinder: **Stüven, Uwe**
**Im Hopfengarten 35**
**D-6232 Bad Soden am Taunus(DE)**
Erfinder: **Sickmüller, Alfred, Dr.**
**Schweinfurter Weg 72**
**D-6000 Frankfurt am Main 70(DE)**
Erfinder: **Wheeler. Larry O., Dr.**
**11122 Jackson Terrace**
**Corpus Christi, TX 78410(US)**
Erfinder: **Moss, Gary L.**
**1113 Mulholland Drive**
**Corpus Christi, TX 78410(US)**
Erfinder: **Zey, Edward G., Dr.**
**522 Evergreen**
**Corpus Christi, TX 78412(US)**
Erfinder: **Voorhoeve, Rudolf J. H., Dr.**
**Hubertusstrasse 2**
**D-6232 Bad Soden am Taunus(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Verfahren zur Herstellung von Arzneimittelwirkstoff-Partikeln mit verbesserten Fliess-, Lager- und Formulierungseigenschaften sowie Arzneimittel, die diese Arzneimittelwirkstoff-Partikeln enthalten.**

(57) Ein Sprühkühlverfahren eignet sich zur Herstellung von Arzneimittelwirkstoff-Partikeln mit verbesserten Fließ-, Lager- und Formulierungseigenschaften.

**Verfahren zur Herstellung von Arzneimittelwirkstoff-Partikeln mit verbesserten Fließ-, Lager- und Formulierungseigenschaften sowie Arzneimittel, die diese Arzneimittelwirkstoff-Partikeln enthalten**

Die vorliegende Erfindung betrifft ein Verfahren zur Herstellung von Arzneimittelwirkstoff-Partikeln mit verbesserten Fließ-, Lager- und Formulierungseigenschaften sowie Arzneimittel, die diese Arzneimittelwirkstoff-Partikeln enthalten.

Viele Arzneimittelwirkstoffe werden zur Herstellung von Arzneimitteldarreichungsformen als Festkörper eingesetzt. Diese Festkörper sind häufig durch eine Umkristallisation gereinigt worden, (vgl. britische Patentschrift GB 971,700, europäische Offenlegungsschrift EP- 170 147 oder rumänische Patentanmeldung 79 345) was dazu führt, daß scharfkantige -häufig nadelförmige -, sich statisch aufladende und zur Zerbrechlichkeit neigende Kristalle erhalten werden. Bei der Herstellung von Arzneimitteldarreichungsformen mit diesen kristallinen Arzneimittelwirkstoffen tritt häufig das Problem auf, daß die Arzneimittelwirkstoffe unter Staubbildung verklumpen oder sich verfestigen, so daß die Arzneimittelwirkstoffe in den betreffenden Herstellungsapparaturen nicht oder unregelmäßig transportiert werden, was u.a. zu Dosierungsschwankungen der Arzneimittel führen kann. Um diese Schwierigkeiten zu vermeiden werden den Arzneimittelwirkstoffen bei der Weiterverarbeitung Hilfsmittel (Zuschlagstoffe) zugesetzt, die eine gute Fließfähigkeit gewährleisten. So ist z.B. bei der maschinellen Herstellung von Ibuprofen-(2-(4-Isobutylphenyl)-propionsäure)-Tabletten der Zusatz von 50 Gew.-% an Hilfsstoffen, bezogen auf das Ibuprofen, notwendig.

Das Zusetzen dieser Hilfsstoffe führt aber andererseits, insbesondere bei hochdosierten Arzneimitteln, z.B. zu Tabletten oder Kapseln, deren Einnahme mit Schluckschwierigkeiten verbunden ist, was sich u.a. negativ auf die "Patientencompliance" auswirken kann.

Überraschenderweise wurde nun gefunden, daß viele Arzneimittelwirkstoffe in eine Form mit verbesserten Fließ-, Lager- und Formulierungseigenschaften überführt werden können, indem man sie einer Sprühkühlung unterwirft. Gegenüber Arzneimittelwirkstoff-Kristallen, die auf herkömmliche Weise durch Umkristallisation aus Lösemitteln erhalten wurden, haben die durch Sprühkühlung erzeugten Arzneimittelwirkstoff-Partikeln meist eine geringere statische Aufladung und eine rundlichere, kompaktere Form. Dies läßt sich am Beispiel Ibuprofen verdeutlichen.

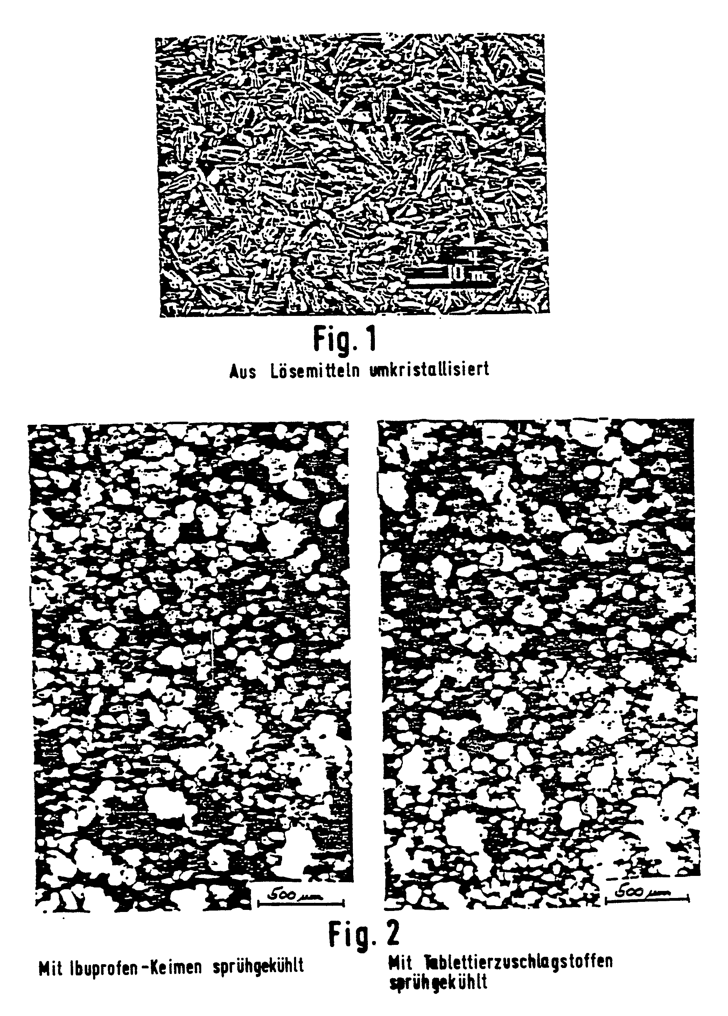

Figur 1 zeigt durch Umkristallisation erhaltene Ibuprofenkristalle, deren nadelförmige Struktur zu Kristallbruch mit Staubbildung und Zusammenbacken zu einem Kristallklumpen neigen.

Figur 2 zeigt Ibuprofen-Partikel, die durch Sprühkühlung einer Ibuprofen-Schmelze erhalten wurden und eine rundliche kompakte Form haben und demzufolge verbesserte Fließ-, Lager- und Formulierungseigenschaften aufweisen.

Dies ist außerordentlich überraschend, weil die bei etwa 74,8° C erstarrende Ibuprofen-Schmelze beim Abkühlen ölig-viskos wird, zur Unterkühlung neigt und nur sehr langsam in den kristallinen Zustand überführt werden kann.

Die erhaltenen Ibuprofen-Partikeln lassen sich z.B. mit nur 15 bis 20 Gew.-% (bezogen auf das Ibuprofen) an Hilfsstoffen zu Tabletten verarbeiten, was die Herstellung von kleinen, leicht schluckbaren Tabletten erlaubt. Die genannten Ibuprofen-Partikeln zeichnen sich weiterhin dadurch aus, daß sie schnell zerfallen (vgl. Bsp. 2), was für die Resorption des Arzneimittels von großer Bedeutung sein kann.

Erfindungsgegenstand ist demzufolge ein Verfahren zur Herstellung von Arzneimittelwirkstoff-Partikeln für die Arzneimittelherstellung, dadurch gekennzeichnet, daß der Arzneimittelwirkstoff einer Sprühkühlung unterzogen wird.

Zur Durchführung des erfindungsgemäßen Verfahrens wird die Arzneimittelwirkstoff-Schmelze in einem Sprühkühlturm geeigneter Konstruktion (vgl. z. B. "A generalised Procedure for the Design and Rating of Prilling Towers", G. L. Wells, J. Kern CIT 51 (1979) 6, 674-675 und CIT MS 702/79) zerstäubt, wobei die Schmelze sowohl von oben (Gleichstrom) als auch von unten (Gegenstrom), vorzugsweise von oben, in den Turm eingebracht werden kann.

Für den Zerstäubungsprozeß werden vorzugsweise Ein- oder Zweistoffdüsen eingesetzt.

Als Kühlgas kommen unterschiedliche Gase in Betracht, vorzuziehen ist als Kühlgas Stickstoff. Der für den Prozeß erforderliche Kühlgasbedarf ist unterschiedlich und hängt u.a. ab von der Temperatur der Schmelze, der Eintrittstemperatur des Gases und dem Festpunkt des jeweiligen Arzneimittelwirkstoffes. Der Kühlgasbedarf bewegt sich bei der Sprühkühlung von Ibuprofen im allgemeinen zwischen 20 und 120 kg, vorzugsweise zwischen 60 und 100 kg Gas pro kg Schmelze. Die Temperatur des Kühlgases wird bei der Sprühkühlung von Ibuprofen beim Gaseintritt vorzugsweise auf 0 bis 50° C, besonders bevorzugt auf 10 bis 30° C, eingeregelt.

Die Sprühkühlung kann bei unterschiedlichen Drücken durchgeführt werden- sowohl bei Normaldruck, als auch bei Überdruck als auch bei Unterdruck - vorzuziehen ist die Durchführung des Verfahrens bei Normaldruck.

Die Sprühkühlung von Arzneimittelwirkstoffen kann mit oder ohne Impfgut (zur Aufhebung von Keimbildungshemmung) vorgenommen werden. Bei Schmelzen, die zur Unterkühlung oder zu langsamem Erstarren neigen, empfiehlt es sich, Impfgut zu verwenden. Das Impfgut kann wahlweise getrennt von der Schmelze in den Turm eingebracht und verteilt werden, es kann in die Schmelze eingearbeitet werden oder es kann sowohl in der Schmelze als auch im Turm vorhanden sein. Als Impfgut eignen sich z.B. Hilfsstoffe oder der Arzneimittelwirkstoff selbst, wobei die Verwendung des Arzneimittelwirkstoffes vorzuziehen ist. Die Menge an zu verwendendem Impfgut hängt ab von dem Arzneimittelwirkstoff, von der Gas-Eintrittstemperatur und von der Turmgeometrie. Bei der Sprühkühlung von Ibuprofen ist ein Impfgut-Zusatz von 10 - 50 % vorzuziehen, besonders bevorzugt ist ein Zusatz von 30 - 35 %, jeweils bezogen auf das Gewicht der Schmelze.

Die durch die Sprühkühlung erhaltenen Arzneimittelwirkstoff-Partikeln können eine unterschiedliche Größe mit einer engen Größenverteilung aufweisen; es ist vorzuziehen, Partikeln mit einem mittleren Durchmesser $d_{50}$ zwischen ca. 20 und 200 $\mu$m, besonders bevorzugt zwischen ca. 60 und 100 $\mu$m, herzustellen. Die Korngröße des Impfgutes kann im gleichen Größenbereich liegen.

Das erfindungsgemäße Verfahren kann für unterschiedliche Arzneimittelwirkstoffe, die sich unzersetzt schmelzen lassen, angewandt werden. Besondere Bedeutung besitzt es für die Konfektionierung von Ibuprofen, insbesondere von Ibuprofen, das durch eine Rektifikation gemäß der deutschen Patentanmeldung P 38 02 619 gereinigt worden ist.

Das wie zuletzt beschrieben gereinigte Ibuprofen kann unmittelbar der Sprühkühlung unterworfen werden, was eine ökonomische Darstellungsmethode für hochwertige Ibuprofen-Partikeln darstellt, unter Vermeidung von Arbeitsschritten mit Beteiligung von Lösemitteln mit allen bekannten Nachteilen wie das Anfallen von Lösemittelabfällen, Lösemittelabluft etc..

Die Weiterverarbeitung der erfindungsgemäß erhaltenen Arzneimittelwirkstoff-Partikeln, die ebenfalls Gegenstand der vorliegenden Erfindung sind, erfolgt in allgemein bekannter Weise, wobei der Hilfsstoffanteil bei der Weiterverarbeitung im Vergleich zum Stand der Technik niedrig gehalten werden kann.

Durch die nachfolgenden Beispiele, die im einzelnen
- die Sprühkühlung der Schmelze und Schmelzgemische mit pharmazeutisch zugelassenen Hilfsstoffen sowie
- die anschließende Tablettierung umfassen, soll die Erfindung näher erläutert werden.


## 1. Beispiel einer Sprühkühlausrüstung

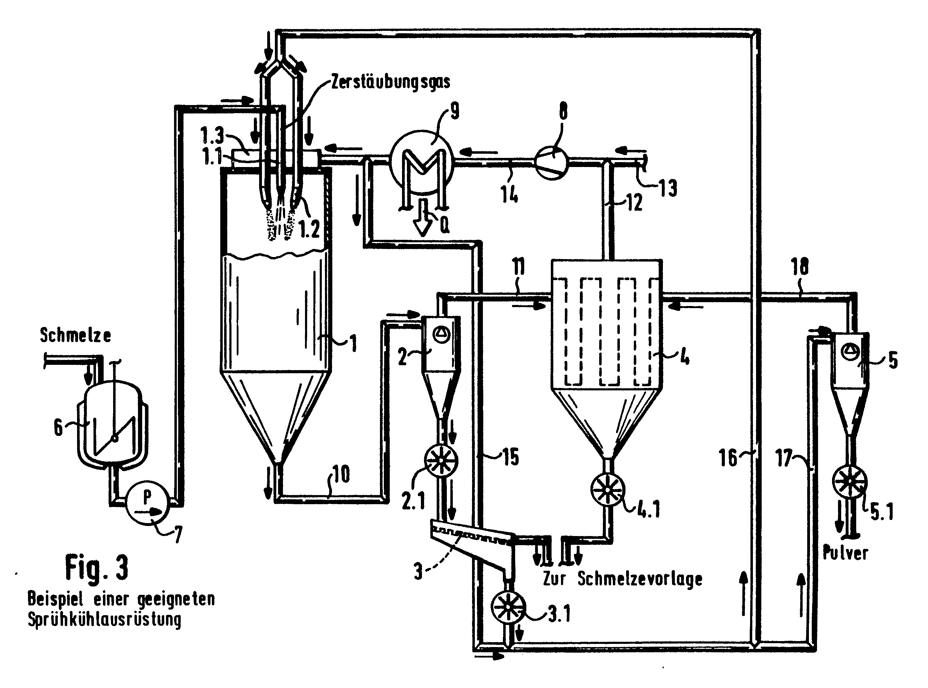

Eine besonders geeignete Sprühkühlausrüstung zeigt Figur 3.

Kernstück ist der Sprühkühlturm 1. Aus der Schmelzevorlage 6 wird über die Pumpe 7 die Zerstäubungsdüse 1.1 beschickt. Beispielhaft ist eine Zweistoffdüse mit zusätzlicher Zufuhr des Zerstäubungsgases dargestellt. Mit Hilfe der Düse 1.2 werden die Kristallisationskeime zugeführt. Als günstig erweist sich die dargestellte Anordnung mit direkter Vermischung im Sprühstrahlbereich. Das Kühlgas tritt bei der gezeigten Gleichstromfahrweise über den Deckengasverteiler 1.3 in den Sprühturm ein. Es verläßt den Turm beladen mit Pulver am Konus und gelangt mittels Leitung 10 zum Zyklonabscheider 2. Der Großteil des Pulvers wird hier abgeschieden und mit Hilfe der Zellenradschleuse 2.1 auf das Sieb 3 ausgetragen. Die Maschenweite des Siebes liegt zwischen 100 $\mu$m und 1 mm, vorzugsweise zwischen 300 $\mu$m und 500 $\mu$m. Die Grobfraktion wird abgetrennt und beispielsweise wieder aufgeschmolzen. Das Feingut wird mittels Durchblasschleuse 3.1 und Inertgas (z.B. Stickstoff) aus Leitung 15 weggefördert. Dabei wird ein entsprechend des Keimbedarfs erforderlicher Strom abgezweigt und über Leitung 16 zu den Puderdüsen 1.2 geblasen, während das Produkt im Zyklon 5 abgeschieden und an der Zellenradschleuse 5.1 ausgetragen wird.

Die jeweils mit nicht abgeschiendenem Feinstaub beladenen Abgasströme aus den Zyklonabscheidern 2 und 5 werden über die Leitungen 11 und 18 in eine Filterkammer oder einen anderen Entstaubungsapparat geführt und dort gereinigt. Das mit der Zellenradschleuse 4.1 ausgetragene Feinstgut kann beispielsweise wieder aufgeschmolzen werden. Der gereinigte Inertgasstrom wird vom Ventilator 8 über Leitung 12 angesaugt, wobei eventuelle Verluste durch Nachspeisung aus Leitung 13 ergänzt werden können. Nach Abfuhr der aufgenommenen Erstarrungswärme Q im Kühler 9 gelangt der Hauptstrom zurück in den Deckengasverteiler 1.3, ein Teilstrom wird über Leitung 15 zur Förderung des Feingutes vom Sieb 3 abgezweigt.

## 2. Anwendungsbeispiel

| Ibuprofen-Tabletten bzw. überzogene Tabletten zu 200 mg Zusammensetzung | |
|---|---|
| | **1 Tablette** |
| 1. Ibuprofen-Pulver, sprühgekühlt | 200 mg |
| 2. Mikrokristalline Cellulose | 30 mg |
| 3. Natriumstärkeglykolat | 2,0 mg |
| 4. Hochdisperses Siliciumdioxid | 1,5 mg |
| 5. Magnesiumstearat | 0,5 mg |

## Herstellung

Die Substanzen 1. - 5. werden in einem geeignten Mischer gemischt und auf einer Rundläufer-Tabletten-Maschine zu Tabletten verpreßt. Falls erwünscht können diese Tabletten in bekannter Weise mit Filmlacklösungen oder -suspensionen auf wäßriger bzw. organischer Basis, z.B. mit Celluloseethern als Filmbildner überzogen werden. Ebenfalls ist ein Überziehen mit Drageesuspensionen auf Saccharosebasis möglich.

## In vitro Wirkstoff-Freigabe (dissolution rate)

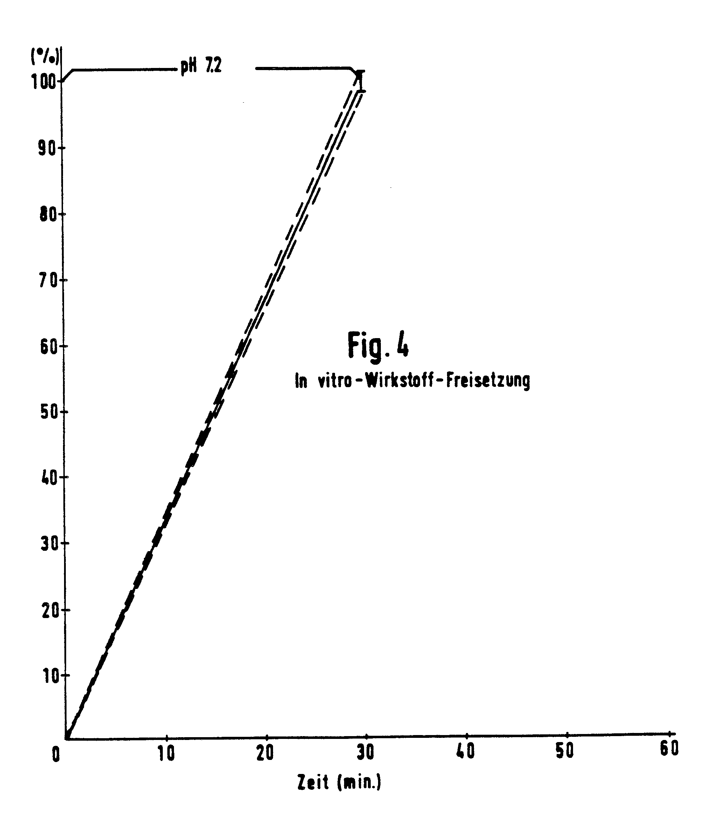

Die "dissolution rate" von Ibuprofen-Tabletten zu 200 mg wurde gemäß den Anforderungen der USP XXI nach folgender Methode bestimmt:
Apparatur 1 (Drehkörbchen-Methode), pH 7,2, Phosphat-Puffer, 900 ml, 150 UpM, Prüfdauer 30 Minuten. Limit nach USP XXI: Q = 50 %. Ergebnis: 100%, vgl. Fig. 4.

## 3. Anwendungsbeispiel

| Ibuprofen-Tabletten bzw. überzogene Tabletten zu 600 mg Zusammensetzung | |
|---|---|
| | **1 Tablette** |
| 1. Ibuprofen-Pulver, sprühgekühlt bestehend aus 80 Gewichtsteilen Ibuprofen und 20 Gewichtsteilen mirkokristalline Cellulose | 750,0 mg |
| 2. Natriumstärkeglykolat | 6,0 mg |
| 3. Hochdisperses Siliciumdioxid | 3,0 mg |
| 4. Magnesiumstearat | 4,0 mg |

## Herstellung

Die Substanzen 1. - 4. werden gemischt und auf einer Rundläufer-Tablettenmaschine zu Tabletten verpreßt.
Falls erwünscht, können die Tabletten wie im Beispiel 1 beschrieben, filmlackiert oder zuckerdragiert werden.

**Ansprüche**

1. Verfahren zur Herstellung von Arzneimittelwirkstoff-Partikeln für die Arzneimittelherstellung, dadurch gekennzeichnet, daß der Arzneimittelwirkstoff einer Sprühkühlung unterzogen wird.

2. Verfahren nach Anspruch 1, dadurch gekennzeichnet, daß man bei der Sprühkühlung Impfgut einsetzt.

3. Verfahren nach Anspruch 2, dadurch gekennzeichnet, daß als Impfgut Arzneimittelwirkstoff-Partikeln eingesetzt werden.

4. Verfahren nach einem oder mehreren der Ansprüche 1 - 3, dadurch gekennzeichnet, daß bei der Sprühkühlung Impfgut in einer Menge von 10 - 50 Gew.-%, bezogen auf die Arzneimittelwirkstoff-Schmelze eingesetzt wird.

5. Verfahren nach einem oder mehreren der Ansprüche 1 - 4, dadurch gekennzeichnet, daß bei der Sprühkühlung der Arzneimittelwirkstoff-Schmelze Tablettier-Zuschlagstoffe zugesetzt werden.

6. Verfahren nach einem oder mehreren der Ansprüche 1 - 5, dadurch gekennzeichnet, daß der Arzneimittelwirkstoff Ibuprofen ist.

7. Verfahren nach einem oder mehreren der Ansprüche 1 - 6, dadurch gekennzeichnet, daß der Arzneimittelwirkstoff Ibuprofen ist, das durch eine Rektifikation gereinigt worden ist.

8. Arzneimittelwirkstoff-Partikeln, dadurch gekennzeichnet, daß sie gemäß einem Verfahren nach einem oder mehreren der Ansprüche 1 - 7 erhalten wurden.

9. Verwendung von Arzneimittelwirkstoff-Partikeln gemäß Anspruch 8, gegebenenfalls unter Zusatz von Hilfs-und/oder Trägerstoffen zur Herstellung von Arzneimittel-Darreichungsformen, insbesondere von Tabletten.

10. Arzneimittel-Darreichungsformen, gekennzeichnet durch einen Gehalt an Arzneimittelwirkstoff-Partikeln gemäß Anspruch 8.

**Fig. 1**

Aus Lösemitteln umkristallisiert

**Fig. 2**

Mit Ibuprofen-Keimen sprühgekühlt

Mit Tablettierzuschlagstoffen sprühgekühlt

Zerstäubungsgas

Schmelze

**Fig. 3**
Beispiel einer geeigneten
Sprühkühlausrüstung

Zur Schmelzevorlage

Pulver

EP 0 362 731 A2

00/F 753

**Fig. 4**
In vitro-Wirkstoff-Freisetzung